# EUROPEAN PATENT SPECIFICATION

(11) **EP 0 770 387 B1**
(45) Date of publication and mention of the grant of the patent: **11.08.1999**
(21) Application number: 95117034.9
(22) Date of filing: 28.10.1995
(51) Int. Cl.: A61K 31/445, A61K 31/165, A61K 31/245, A61K 9/107, A61K 31/485

(54) **Pharmaceutical composition containing a local anesthetic and/or centrally acting analgesic**
Lokalanästhetika und/oder Zentralanalgetika enthaltende pharmazeutische Zusammensetzungen
Composition pharmaceutique contenant un anesthésique locale et/ou un analgésique à action centrale

(43) Date of publication of application: 02.05.1997
(73) Proprietor: B. BRAUN MELSUNGEN AG, 34209 Melsungen (DE)
(72) Inventor: Toledo, Alfonso, E-08025 Barcelona (ES)
(74) Representative: Weber, Thomas, Dr.Dipl.-Chem.

(56) References cited:
- EP-A- 0 391 369
- WO-A-93/19736
- US-A- 4 168 308
- US-A- 5 227 165
- ACTA PHARMACOLOGICA ET TOXICOLOGICA, vol. 36, no. 4, 1975, pages 299-311, XP002000450 R. JEPPSSON: "Comparison of pharmacological effects of some local anaesthetic agents when using water and lipid emulsion as injection vehicles."
- J. PARENTER. SCI. TECHNOL., vol. 41, no. 6, 1987, pages 220-224, XP002000451 R.T. LOSTRITTO: "Temperature and cosurfactant effects on lidocaine release from submicron oil in water emulsions"
- J. PHARM. SCI., vol. 63, no. 9, 1974, pages 1402-1406, XP002000452 J.W. AYRES: "Diffusion of benzocaine from ointment bases."

## Description

The present invention relates to novel pharmaceutical compositions for the regional anesthesia.

Local anesthetics are compounds which reversibly block impulse conduction along nerve axons. This action is used clinically to provide temporary analgesia in specific areas of the body after injection in the vicinity of peripheral nerve endings, major nerve trunks, or spaces surrounding the spinal cord.

A recognized object to the use of local anesthetics is their limited duration of action when administered as a single injection. Continuous or repeated administration requires catheterization which is technically complicated, time consuming, expensive, and may cause several complications. Furthermore, when local anesthetics are administered in successive injections, each dose is less effective than its predecessor. Prolongation of the anesthesia increasing the dose of the anesthetic is not recommended because this may cause very serious or even irremediable toxic effects as a result of the raised plasma concentrations. The systemic toxic effects mainly involves the central nervous system (dizziness, tinnitus, tremors, and convulsions) and the cardiovascular system (hypertension, brachycardia, arrhythmias, and cardiac arrest).

Several attempts have been made to implement slow-delivery drug systems for local anesthetics.

Polymer matrixes implants are described in patents WO-A-93/20138, WO-A-92/13567 and US-A-5 179 189. Although very prolonged regional nerve blockade can be achieved with these implants (Masters D.B. et al., Anesthesiology 1993, 79, 340-346) their application is limited to the treatment of chronic pain.

Injectable formulations of local anesthetics containing organic solvents (WO-A-94/01087), polylactic acid microspheres (Wakiyama N. et. al., Chem-Pharm.Bull. 1981, 29(11), 3363-3368) and solid lipospheres (WO-A-91/07171) have been proposed, but the clinical usefulness of the aforementioned systems has hitherto not been proved.

A formulation prepared by dissolving hydrophobic drugs (local anesthetics and opiates) in iophendylate (a contrast medium used for X-ray examination) has been proposed as injectable slow-release system for spinal anesthesia (IL-A-91597). Iophendylate preparations of local anesthetics produce prolonged but less intense nerve blockade than the aqueous solutions (Langermann L. et al., Anesthesiology 1992, 77, 475-481). Furthermore, severe toxic reactions (arachnoiditis and aseptic meningitis) have been reported after intrathecal injection of iophendylate.

Liposomes have been widely used as drug carriers. In EP-A-0233 100 encapsulation of local anesthetics into liposomes is proposed. "In vivo" experiments with liposome-encapsulated lidocaine or bupivacaine demonstrated slow-release pharmacokinetic profiles and prolongation of nerve blocking effect without side effects (Legros F. et al., Anesthesiology 1990, 73, A851; Mashimo T. et al., Anesth. Analg. 1992, 74, 827-834). However, large-scale manufacture of liposome products presents a variety of challenges: solvent incompatibility with process equipment, particle size distribution, material stability during processing, drug entrapment consistency, sterile product production, and free drug, solvent, and detergent removal (Weiner A.L. Pharm. Technol. Int., June 1990, 46-50). Another major potential obstacle to the use of liposomes as pharmaceuticals is their instability during extended storage (Ostro M.J. et al., Amer. J. Hosp. Pharm. 1989, 46, 1576-1587). Moreover, local anesthetics increase the fragility of liposomes (Surewicz W.K. et. al., Biochem-Pharmacol. 1982, 31, 2999-3000).

Oil-in-water (o/w) emulsions have been widely investigated as a vehicle for parenteral administration of water-insoluble drugs (see, for example patents US-A-4,073, 943 and US-A-4,168,308). Some of the advantages of emulsions systems over other particulate carriers, such as liposomes and microparticles are: they may be biodegradable; technologies exist for their large-scale production; they have satisfactory shelf life; and they are already clinically acceptable. Parenteral products containing emulsified vegetable oils have been in clinical use for nearly 30 years and emulsions containing water-insoluble drugs are already either in clinical trials or are being marketed. These reasons have lead to the examination of the possibility of prolonging local anesthesia by using o/w emulsions as drug carrier.

EP-A-0 391 369 discloses an oil-in-water emulsion containing a hydrophobic drug comprising
(i) an oily carrier of MCT or a mixture of MCT/vegetable oil (LCT);
(ii) phospholipids as emulsifier;
(iii) a non-ionic surfactant; and
(iv) an ionic surfactant selected from a bile-duct surface active agent cholic acid and deoxycholic acid, and their surface active derivatives and salts.

The combination of the non-ionic surfactant and the ionic surfactant as essential ingredients is employed to better solubilize the hydrophobic drug in the oily carrier. In the very sensible area of anesthetics, for instance peridural anesthesy, it is not desirable to use non-ionic surfactants as they may cause unpredictable severe physiological side-effects.

US-A-4,168,308 describes the incorporation of a great number of pharmacologically active agents, including local anesthetics, into vegetable oils emulsified with poloxamer and acetylated monoglycerides.

The publication of Acta Pharmacol. Toxicol. 36, 299-311 (1975) shows that the duration of the anesthetic effect of the afore-mentioned o/w emulsions is not significantly increased compared the aqueous solutions, and in some instance the inclusion of the anesthetic in the emulsion completely abolishes the pharmacologic effect. Thus, the prior art has not given positive results in the preparation of an useful, stable, effective, safe, and non-toxic injectable formulation of local anesthetics analgesics with longer-lasting activity and lower potential toxicity.

It would therefore be desirable to have available controlled-release formulations of local anesthetics which allow to overcome the main drawbacks to use local anesthetics: short duration and systemic toxicity.

It was therefore an object of the present invention to provide a long-term stable emulsion formulation having prolonged local anesthetic/analgesic activity and lower systemic toxicity.

The present invention proposes pharmaceutical compositions containing local anesthetics and/or centrally acting analgesics, which are substantially encapsulated in the oil droplets of an o/w emulsion.

In detail the present invention relates to a pharmaceutical composition in the form of an oil-in-water emulsion (o/w) consisting essentially of
(a) 5 to 30% (w/v) of an oily carrier consisting of long-chain triglycerides (LCT) and/or medium-chain triglycerides (MCT),
(b) 0.5 to 2% (w/v) of an emulsifier,
(c) 0.1 to 2% (w/v) of a local anesthetic and/or centrally acting analgesic,
(d) conventional additives,
   under the proviso that the composition does not contain non-ionic surfactants.

Throughout the present specification the indication of % (percent) is intended to mean w/v, i.e. weight of ingredient per volume of the entire aqueous emulsions.

The long-chain triglycerides (LCT) are triglycerides of saturated and/or unsaturated fatty acids containing 16 to 22, preferably 16 to 18 carbon atoms. Specific examples are soybean oil, olive oil, cottonseed oil and sesame oil which contain as a major part said LCT's.

The medium-chain triglycerides (MCT) are triglycerides of saturated and/or unsaturated fatty acids having 8 to 12 carbon atoms. Specific examples are Miglyol (Hüls AG, Germany) and TCM (Societé des Oleaginaux, France).

Suitable emulsifiers which are used according to the present invention are selected from phospholipids such as egg yolk phospholipids (egg yolk lecithin), soybean oil phospholipids (soybean lecithin) or mixtures thereof, which are available from commercial sources.

In the final emulsion, the total proportion of oily carrier (consisting of MCT and/or LCT) is suitably 5 to 30% (w/v), preferably 10 to 20% (w/v). If mixtures of LCT and MCT are used a suitable LCT/MCT weight ratio is 10:1 to 1:10, preferably 5:1 to 1:5, most preferred 1:1.

The emulsifier is preferably used in an amount of 1 to 2% (w/v).

The level of the drug is preferably 0.125% (w/v) to 1.0% (w/v).

The local anesthetic is selected from benzoic acid derivatives, anilide derivatives and cocaine derivatives. Preferred anesthetics are selected from tetracaine, benzocaine, procaine, butylaminobenzoate, bupivacaine, ropivacaine, mepivacaine, lidocaine, prilocaine and cocaine.

The centrally acting analgesic is selected from morphine or morphine derivatives, phenylheptylamine derivatives or phenyl piperidine derivatives. Preferred centrally acting analgesics are selected from morphine hydromorphone, oxymorphone, methadone, meperidine, alfentanil, fentanyl and sufentanil.

In many cases combination of a local anesthetic and a centrally acting analgesic are used for practical purposes.

In the emulsion according to the present invention at least 98%, preferably at least 99% of the drug is encapsulated into the oil droplets.

The emulsion may also contain conventional emulsion additives in conventional amounts, such as emulsifying aids, tonicity agents and antioxidants.

As additional emulsifying aid, the emulsion may also contain sodium salts of long-chain fatty acids. These coemulsifiers may be salts of palmitic acid, stearic acid, oleic acid, linoleic acid or linolenic acid. Said salts are used in an amount of up to 5% (w/v) of the emulsion composition, preferably in an amount between 0.005% (w/v) to 0.1% (w/v).

If necessary, tonicity can be adjusted with an agent that does not adversely affect emulsion stability (i.e. non electrolyte) glycerol, glucose, mannitol, sorbitol or xylitol are suitable choices, glycerol being preferred.

The compositions may also contain an antioxidant such as α-tocopherol or its ester.

The pharmaceutical compositions of the present invention contain no non-ionic surfactants alone or a combination thereof with other types of surfactants.

The active principle, in the form of the liposoluble base, is dissolved in the oil mixture, and the emulsion is prepared according to known methods.

The aqueous solution and the oily solution comprising the drugs as liposoluble base are then mixed with one another. However, the so-obtained mixture does not yet consist of sufficiently small droplets, the size of which (obtained after mixing with a magnetic stirrer) is about 10 µm. The droplet size of the inventive composition may then be decreased by the use of emulsification equipment such as Ultra Turrax (Jankl and Kunkel, Staufen, FRG), which yields droplets having an average diameter of about 1 to 1.5 µm, or of a high shear mixer, e.g. Polytron (Kinematica, Lucerne, Switzerland) which yields droplets having an average diameter of about 0.6 to 0.8 µm.

Especially small droplets are obtained in the inventive compositions when utilizing a two-stage pressure homogenizer in which the crude dispersion is forced under high pressure through the annular space between a spring loaded valve and the valve seat, the second stage being in tandem with the first so that the emulsion is subjected to two very rapid dispersion processes. An example of such an apparatus is the Gaulin Homogenizer (APV Gaulin, Hilversum, The Netherlands). Use of such an apparatus in accordance with the invention yields emulsions of the present invention in which the droplets have an average diameter of less than 0.35 µm, preferably between 0.2 µm and 0.35 µm with a relative small deviation.

The resultant emulsion is heat-sterilized according to procedures generally known in the art.

The experiments carried out have demonstrated that the anesthetics/analgesics incorporated in o/w emulsions prepared according to the invention have longer-lasting action and lower plasma levels than the conventional aqueous solution at the equal dose. Thus, an increase in the anesthetic action and a decrease in the side effects can be achieved. Furthermore, it has been found that the pharmaceutical emulsions according to the present invention have a superior long-term stability. Moreover, the emulsions of the present invention are stable to standard autoclave sterilization without changing its physical or chemical properties.

The figures and examples below are given for guidance, and allow explain other characteristics and advantages of the present invention by way of a composition comprising bupivacaine as an active ingredient.
Figure 1 shows the time course of sensory blockade after the injection near the rat sciatic nerve of a bupivacaine composition in accordance with the invention versus the conventional bupivacaine aqueous solution at equivalent doses.
Figure 2 is comparable to Figure 1 and illustrated to motor blockade.
Figure 3 shows the blood pharmacokinetic profile after the administration near the rat sciatic nerve of a bupivacaine composition in accordance with the invention versus the conventional bupivacaine aqueous solution at equivalent doses.
Figure 4 shows the time course of sensory blockade after epidural injection in rats of a bupivacaine composition in accordance with the invention versus the conventional bupivacaine aqueous solution at equivalent doses.
Figure 5 is comparable to Figure 4 and illustrated the abolition of the contralateral extensor reflex response.
Figure 6 shows the blood pharmacokinetic profile after the epidural injection to dogs of a bupivacaine composition in accordance with the invention versus the conventional bupivacaine aqueous solution at equivalent doses.

### EXAMPLES

### EXAMPLE 1 Preparation of the emulsion

An injectable submicron emulsion according to the invention, with a bupivacaine concentration equivalent to bupivacaine hydrochloride 0.5% (w/v), is prepared by known techniques.

### Composition:

10 g soya bean oil
10 g miglyol
1.2 g purified egg yolk lecithin
2.5 g glycerol
0.03 g sodium oleate
0.4439 g bupivacaine base
water to 100 ml

Soya bean oil and miglyol are mixed, and bupivacaine is dissolved in the oil mixture. Sodium oleate, glycerol, and lecithin are added to water. Aqueous and oily phases are thoroughly stirred. The crude dispersion is homogenized by using a two stage high pressure homogenizer. A filtration step is included to remove poorly emulsified material and foreign particles. A standard autoclave sterilization cycle (121 °C, 20 minutes) can be applied without changes in the physical and chemical properties of the emulsion.

### EXAMPLE 2 Preparation of the emulsion

An injectable submicron emulsion according to the invention, with a bupivacaine concentration equivalent to bupivacaine hydrochloride 0.5% (w/v), is prepared by known techniques.

### Composition:

10 g soya bean oil
10 g miglyol
1.2 g purified egg yolk lecithin
0.03 g sodium oleate
0.4439 g bupivacaine base
water to 100 ml

Soya bean oil and miglyol are mixed, and bupivacaine is dissolved in the oil mixture. Sodium oleate, and lecithin are added to water. Aqueous and oily phases are thoroughly stirred. The crude dispersion is homogenized by using a two state high pressure homogenizer. A filtration step is included to remove poorly emulsified material and foreign particles. A standard autoclave sterilization cycle (121 °C, 20 minutes) can be applied without changes in the physical and chemical properties of the emulsion.

### EXAMPLE 3 Preparation of the emulsion

An injectable submicron emulsion according to the invention, with a bupivacaine concentration equivalent to bupivacaine hydrochloride 0.5% (w/v), is prepared by known techniques.

### Composition:

20 g soya bean oil
1.2 g purified egg yolk lecithin
2.5 g glycerol
0.03 g sodium oleate
0.4439 g bupivacaine base
water to 100 ml

Bupivacaine is dissolved in the soya bean oil. Sodium oleate, glycerol and lecithin are added to water. Aqueous and oily phases are thoroughly stirred. The crude dispersion is homogenized by using a two stage high pressure homogenizer. A filtration step is included to remove poorly emulsified material and foreign particles. A standard autoclave sterilization cycle (121 °C, 20 minutes) can be applied without changes in the physical and chemical properties of the emulsion.

### EXAMPLE 4 Preparation of the emulsion

An injectable submicron emulsion according to the invention, with a bupivacaine concentration equivalent to bupivacaine hydrochloride 0.5% (w/v), is prepared by known techniques.

### Composition:

20 g miglyol
1.2 g purified egg yolk lecithin
2.5 g glycerol
0.03 g sodium oleate
0.4439 g bupivacaine base
water to 100 ml

Bupivacaine is dissolved in miglyol. Sodium oleate, glycerol and lecithin are added to water. Aqueous and oily phases are thoroughly stirred. The crude dispersion is homogenized by using a two stage high pressure homogenizer. A filtration step is included to remove poorly emulsified material and foreign particles. A standard autoclave sterilization cycle (121 °C, 20 minutes) can be applied without changes in the physical and chemical properties of the emulsion.

### EXAMPLE 5 Percentage of drug encapsulated into the oil droplets

The percentage of bupivacaine encapsulated into the oil droplets of an emulsion prepared as described in Example 1 was evaluated by ultrafiltration in a Ultrasart Cell (Sartorius GmbH) equipped with a cellulose triacetate ultra-filter (nominal cutoff: molecular wight 10 000). Bupivacaine concentration in the filtrate was determined by high performance liquid chromatography. The quantity of bupivacaine encapsulated into the oil droplets was calculated by subtracting the amount of free bupivacaine in the filtrate from the total amount of drug. The results obtained show that 99.0-99.8% (w/v) of total bupivacaine is in the oil droplets.

### EXAMPLE 6 Long-term stability

Samples of an emulsion prepared as described in Example 1 were stored at 25 °C for one year. Every six months the following parameters were analyzed:
Long-chain triglycerides content
Medium-chain triglycerides content
Glycerol content
Bupivacaine content
Droplet size
Acidity index
Peroxide index
pH
Percentage of bupivacaine encapsulation
Macroscopic appearance.

After 18 months all parameters comply with the specifications stablished for intravenous emulsions. It is noteworthy that the percentage of bupivacaine encapsulated into the oil droplets is maintained above 98% (w/v) of the total amount of the drug.

### EXAMPLE 7 Anesthetic/analgesic effect of a composition according to the invention after injection near the rat sciatic nerve

Ten Sprague-Dawley rats per group were treated with 0.2 ml of an emulsion prepared as described in Example 1 or, by way of control, the same dose of bupivacaine in conventional aqueous solution. The injection site was the space surrounding the sciatic nerve in the popliteal region. Sensory and motor blockades were assessed by recording sensory nerve action potentials (NAP) and compound evoked motor action potentials (CMAP), respectively. NAPs and CMAPs were elicited simultaneously with supramaximal electrical stimulation of the sciatic nerve proximal to the site of injection.

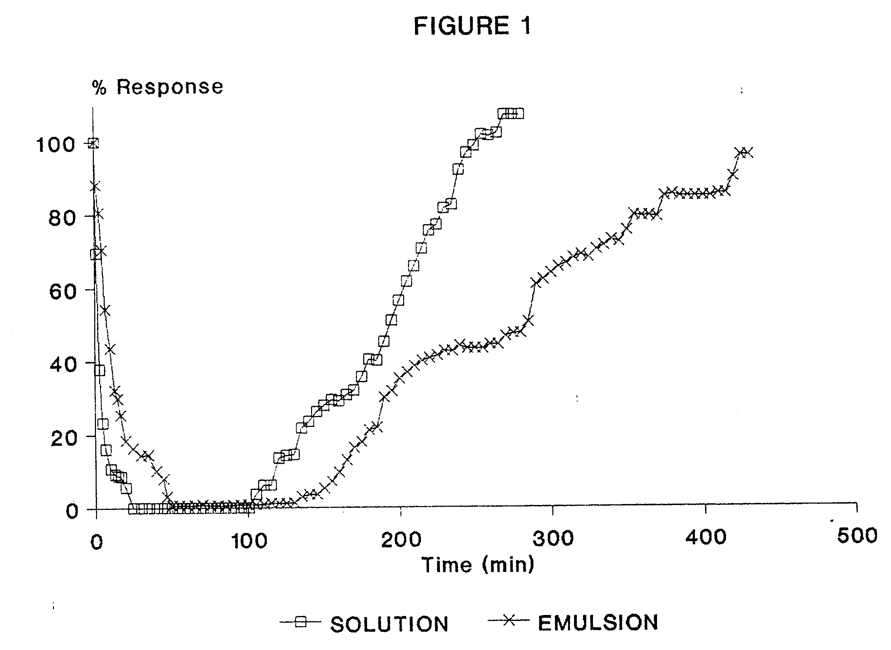

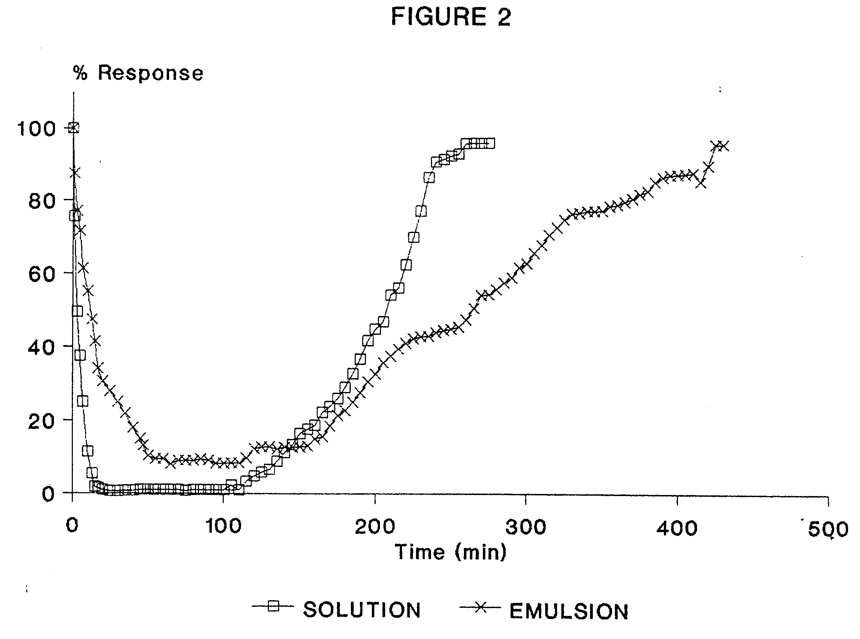

The time-course of mean changes in NAPs and CMAPs are illustrated in Figures 1 and 2. Results are expressed as % (w/v) of pre-injection baseline values.

Bupivacaine incorporated into an emulsion in accordance with the invention significantly (p < 0.05, Mann-Whitney test) increased the duration of total sensory blockade (238.1 ± 81.1 minutes) when compared to aqueous solution (165.0 ± 47.4 minutes). Moreover the recovery period (time until baseline value was reached) was significantly (p < 0.05) more lengthy when bupivacaine was administered into the emulsion 302.5 ± 78.1) than when the aqueous solution was injected (212.2 ± 42.1 minutes).

In the same way, the emulsion significantly (p < 0.05) increased the duration of total motor blockade from 140.6 ± 38.8 to 200.0 ± 77.9 minutes and the recovery period from 218.3 ± 27.0 to 303.1 ± 89.6 minutes, when compared to the aqueous solution.

These results demonstrate that bupivacaine incorporated in an emulsion in accordance with the invention produces, at equal dose, a longer-lasting anesthetic/analgesic action than the conventional aqueous solution.

### EXAMPLE 8 Pharmacokinetics of bupivacaine contained in a composition according to the invention, after injection near the rat sciatic nerve

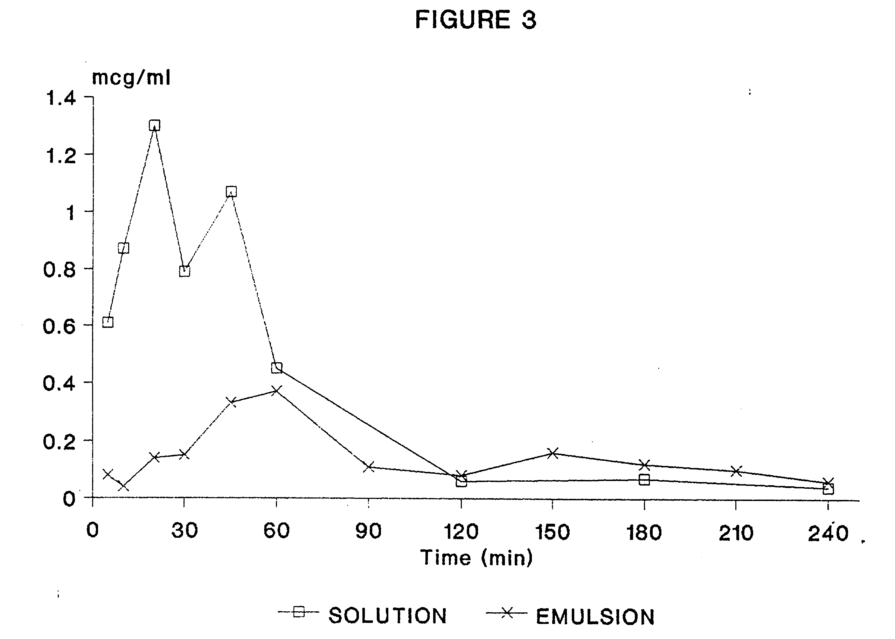

Two groups of Sprague-Dawley rats were treated as described in Example 7. At different times after the injection four rats of each group were sacrified in order to obtain blood samples, and plasma bupivacaine levels were determined. The mean plasma bupivacaine concentrations obtained are shown in Figure 3. Following the injection of bupivacaine incorporated into the emulsion the highest plasma concentration (0.37 ± 0.08 µg/ml) is significantly lower (p < 0.05, Mann-Whitney test) than following the injection of conventional bupivacaine aqueous solution (1.30 ± 0.13 µg/ml). Moreover, the maximum plasma levels are attained at 60 and 20 minutes for emulsion and aqueous solution, respectively. These results indicate that bupivacaine incorporated into an emulsion according to the invention is absorbed toward the bloodstream at lower rate than the conventional aqueous solution.

As the systemic toxic side effects depend on the plasma bupivacaine levels, bupivacaine incorporated into an emulsion according to the invention possesses, at the same dose, greater safety than that of the conventional aqueous solution in the peripheral nerve anesthesia.

### EXAMPLE 9 Anesthetic/analgesic effect of a composition according to the invention, administered epidurally to rats

Ten Sprague-Dawley rats per group were treated with 0.2 ml of an emulsion prepared as described in Example 1 or, by way of control, the same dose of bupivacaine in conventional aqueous solution. The injections were performed introducing a needle through the skin, at the level of L5-L6 lumbar space, until the epidural space was reached. This space is located between the dura-mater (the outermost membrane surrounding the spinal cord) and the vertebral canal. In order to assess the anesthetic/analgesic effect two tests were carried out.

### 1. Nociceptive response:

Electrical stimulus were applied to the hind paw plantar surface. Normal response consist of a general muscular contraction, especially evident in the lumbar and abdominal musculature. The response to the stimulus was semiquantitatively evaluated in the scale: 2 = clear and bilateral contraction; 1 = weak and unilateral contraction; 0 = no response.

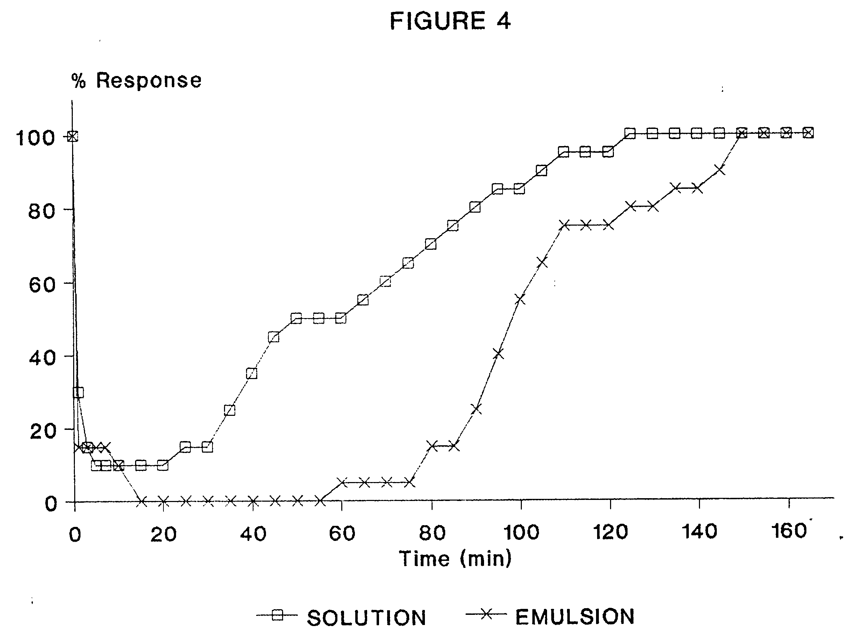

The time-course of mean changes in the nociceptive response are illustrated in Figure 4. Bupivacaine incorporated into the emulsion significantly (p < 0.05, Mann-Whitney test) increased the duration of maximum blockade from 56.0 ± 26.9 to 90.5 ± 18.9 minutes and the recovery period from 72.0 ± 30.2 to 116.5 ± 25.1 minutes, when compared to conventional aqueous solution.

### 2. Extensor reflex response:

Electrical stimulus were applied at the level of the hind paw tibial nerve. The response were electromiografically assessed recording the muscular action potentials in the quadriceps extensor muscle of the contralateral paw.

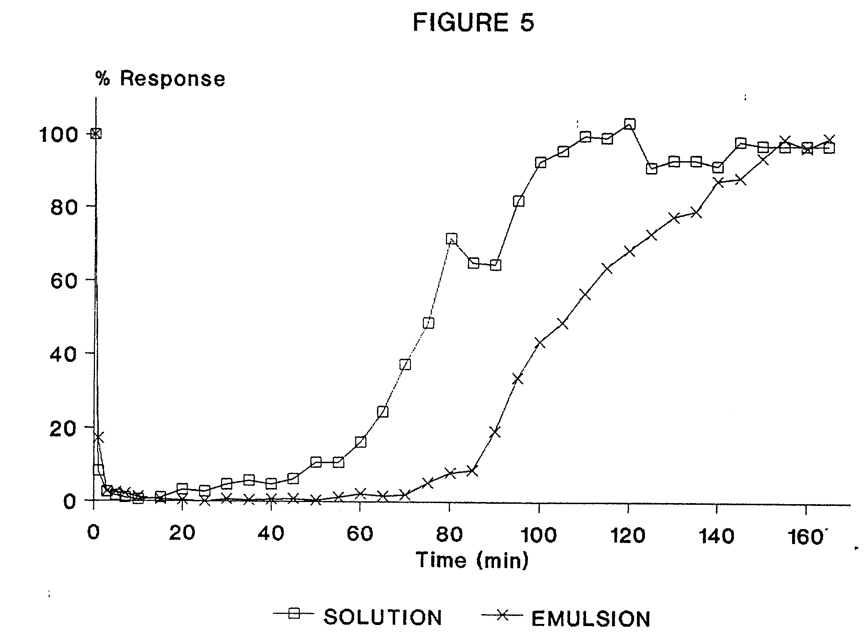

The time course of mean changes in action potentials are illustrated in Figure 5. Results are expressed as % (w/v) of pre-injection base line. Bupivacaine incorporated into the emulsion significantly (p < 0.05) increased the duration of maximum blockade from 49.5 ± 18.6 to 85.0 ± 17.8 minutes and the recovery period from 92.5 ± 25.3 to 128.0 ± 18.5 minutes, when compared to conventional aqueous solution.

These results prove that bupivacaine incorporated into an emulsion according to the invention produces, at equal dose, longer-lasting anesthetic/analgesic action than the conventional aqueous solution in the epidural anesthesia.

### EXAMPLE 10 Pharmacokinetics of bupivacaine contained in a composition according to the invention, administered epidurally to dogs

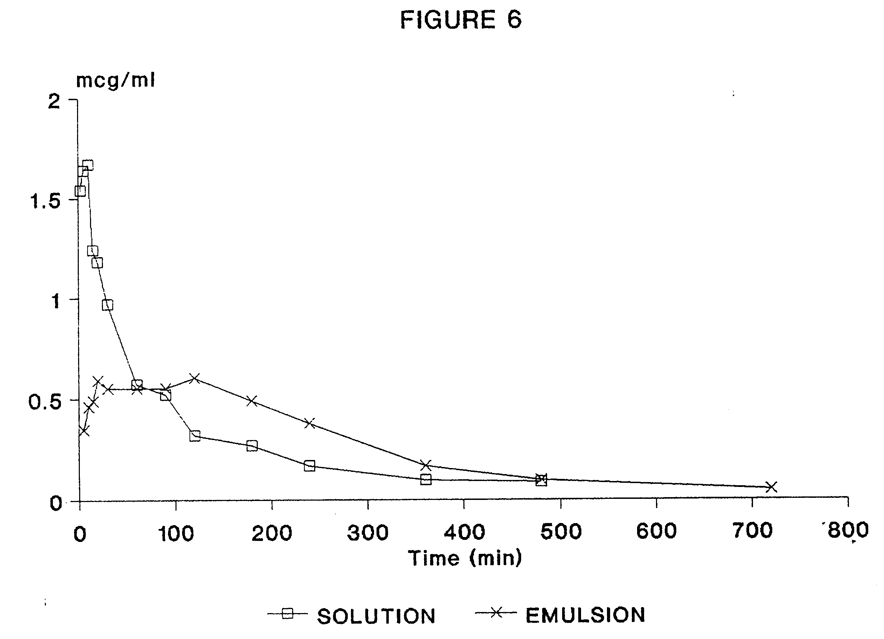

Six Beagle dogs were used. Three dogs were epidurally treated with bupivacaine (1.8 mg/kg) incorporated into an emulsion prepared as described in Example 1 and after ten days with the same dose of bupivacaine in conventional aqueous solution. Three dogs first received bupivacaine in the form of conventional aqueous solution and after ten days bupivacaine in emulsion. After each epidural injection blood samples were collected at different times, and plasma bupivacaine levels were determined. The time-course of mean bupivacaine plasma levels is illustrated in Figure 6. The peak plasma concentration (Cmax) after the epidural administration of bupivacaine in emulsion (0.62 ± 0.24 µg/ml) was significantly lower (p < 0.05, Mann-Whitney test) than the peak plasma concentration after the epidural administration of the conventional aqueous solution (2.00 ± 0.74 µg/ml). The times (tmax) at which these peaks were reached were significantly (p < 0.05) different to: 68.33 ± 33.71 and 6.00 ± 5.40 minutes for the emulsion and the aqueous solution respectively.

As in the peripheral nerve anesthesia (see Example 5), the results obtained in the epidural anesthesia demonstrate that the passage of bupivacaine incorporated into an emulsion according to the invention from the epidural space toward the bloodstream is a slower process than that of bupivacaine in the conventional aqueous solution. In this way, plasma bupivacaine concentration can be maintained far below the toxic levels. Therefore, in the epidural anesthesia, bupivacaine incorporated into an emulsion according to the invention is a safer pharmaceutical preparation than the conventional aqueous solution.

### EXAMPLE 11 Neurotoxicity of a composition according to the invention after subarachnoid injection

Although during the anesthetic/analgesic activity studies no signs of toxicity were observed and complete recovery of sensory and motor functions was achieved, a study was performed to establish possible histopathological differences, if any, among two groups of rats receiving respectively subarachnoid injections of bupivacaine in emulsion or conventional bupivacaine aqueous solution. The administration route chosen (subarachnoid injection) allow the direct contact of bupivacaine preparations with the spinal cord nerve fibers. No appreciable differences were observed among the two groups seven days after the injections.

## Claims

1. A pharmaceutical composition in the form of an oil-in-water emulsion (o/w) consisting essentially of
(a) 5 to 30% (w/v) of an oily carrier consisting of long-chain triglycerides (LCT) and/or medium-chain triglycerides (MCT),
(b) 0.5 to 2% (w/v) of an emulsifier,
(c) 0.1 to 2% (w/v) of a local anesthetic and/or centrally acting analgesic,
(d) conventional additives
under the proviso that the composition does not contain non-ionic surfactants.

2. The pharmaceutical composition of claim 1, wherein the LCT's are selected from triglycerides of saturated and/or unsaturated fatty acids containing 16 to 22 carbon atoms.

3. The pharmaceutical composition of claim 1, wherein the MCT's are selected from triglycerides of saturated and/or unsaturated fatty acids containing 8 to 12 carbon atoms.

4. The pharmaceutical composition of claims 1 to 3, wherein the ratio of LCT:MCT is from 10:1 to 1:10.

5. The pharmaceutical composition of claims 1 to 4, wherein the emulsifier is selected from phospholipids.

6. The pharmaceutical composition of claims 1 to 5, wherein the local anesthetic is selected from benzoic acid derivatives, anilide derivatives, or cocaine derivatives.

7. The pharmaceutical composition according to claim 6, wherein the local anesthetic is selected from tetracaine, benzocaine, procaine, butylaminobenzoate, bupivacaine, ropivacaine, mepivacaine, lidocaine, prilocaine and cocaine.

8. The pharmaceutical composition of claims 1 to 5, wherein the centrally acting analgesic is selected from morphine or morphine derivatives, phenylheptylamine derivatives or phenyl piperidine derivatives.

9. The pharmaceutical composition according to claim 8, wherein the centrally acting analgesic is selected from morphine, hydromorphone, oxymorphone, methadone, meperidine, alfentanil, fentanyl and sufentanil.

## Patentansprüche

1. Pharmazeutische Zusammensetzung in Form einer Öl-in-Wasser-Emulsion (o/w), im wesentlichen bestehend aus:
(a) 5 bis 30% (w/v) eines öligen Trägers, der aus langkettigen Triglyceriden (LCT) und/oder mittelkettigen Triglyceriden (MCT) besteht;
(b) 0,5 bis 2% (w/v) eines Emulgators;
(c) 0,1 bis 2% (w/v) eines Lokalanästhetikums und/oder zentral wirkenden Analgetikums;
(d) herkömmlichen Additiven;
mit der Maßgabe, daß die Zusammensetzung keine nichtionischen Tenside enthält.

2. Pharmazeutische Zusammensetzung gemäß Anspruch 1, wobei die LCTs aus Triglyceriden gesättigter und/oder ungesättigter Fettsäuren mit 16 bis 22 Kohlenstoffatomen ausgewählt sind.

3. Pharmazeutische Zusammensetzung gemäß Anspruch 1, wobei die MCTs aus Triglyceriden gesättigter und/oder ungesättigter Fettsäuren mit 8 bis 12 Kohlenstoffatomen ausgewählt sind.

4. Pharmazeutische Zusammensetzung gemäß Anspruch 1 bis 3, wobei das Verhältnis von LCT:MCT 10:1 bis 1:10 beträgt.

5. Pharmazeutische Zusammensetzung gemäß Anspruch 1 bis 4, wobei der Emulgator aus Phospholipiden ausgewählt ist.

6. Pharmazeutische Zusammensetzung gemäß Anspruch 1 bis 5, wobei das Lokalanästhetikum aus Benzoesäurederivaten, Anilidderivaten oder Kokainderivaten ausgewählt ist.

7. Pharmazeutische Zusammensetzung gemäß Anspruch 6, wobei das Lokalanästhetikum aus Tetracain, Benzocain, Procain, Butylaminobenzoat, Bupivacain, Ropivacain, Mepivacain, Lidocain, Prilocain und Kokain ausgewählt ist.

8. Pharmazeutische Zusammensetzung gemäß Anspruch 1 bis 5, wobei das zentral wirkende Analgetikum aus Morphin oder Morphinderivaten, Phenylheptylaminderivaten oder Phenylpiperidinderivaten ausgewählt ist.

9. Pharmazeutische Zusammensetzung gemäß Anspruch 8, wobei das zentral wirkende Analgetikum aus Morphin, Hydromorphon, Oxymorphon, Methadon, Meperidin, Alfentanil, Fentanyl und Sufentanil ausgewählt ist.

## Revendications

1. Composition pharmaceutique sous forme d'une émulsion d'huile dans l'eau (h/e) constituée essentiellement
(a) de 5 à 30% (p/v) d'un véhicule huileux constitué de triglycérides à chaîne longue (TCL) et/ou de triglycérides à chaîne moyenne (TCM),
(b) de 0,5 à 2% (p/v) d'un émulsifiant,
(c) de 0,1 à 2% (p/v) d'un anesthésique local et/ou d'un analgésique d'action centrale,
(d) d'additifs classiques
à condition que la composition ne contienne pas de tensioactifs non ioniques.

2. Composition pharmaceutique selon la revendication 1, dans laquelle les TCL sont choisis parmi les tricycérides d'acides gras saturés et/ou insaturés contenant 16 à 22 atomes de carbone.

3. Composition pharmaceutique selon la revendication 1, dans laquelle les TCM sont choisis parmi les tricycérides d'acides gras saturés et/ou insaturés contenant 8 à 12 atomes de carbone.

4. Composition pharmaceutique selon les revendications 1 à 3, dans laquelle le rapport de TCL/TCM est de 10:1 à 1:10.

5. Composition pharmaceutique selon les revendications 1 à 4, dans laquelle l'émulsifiant est choisi parmi les phospholipides.

6. Composition pharmaceutique selon les revendications 1 à 5, dans laquelle l'anesthésique local est choisi parmi les dérivés d'acide benzoïque, les dérivés d'anilide ou les dérivés de cocaïne.

7. Composition pharmaceutique selon la revendication 6, dans laquelle l'anesthésique local est choisi parmi la tétracaïne, la benzocaïne, la procaïne, l'aminobenzoate de butyle, la bupivacaïne, la ropivacaïne, la mépivacaïne, la lidocaïne, la prilocaïne et la cocaïne.

8. Composition pharmaceutique selon les revendications 1 à 5, dans laquelle l'analgésique d'action centrale est choisi parmi la morphine ou les dérivés de morphine, les dérivés de phénylheptylamine ou les dérivés de phénylpipéridine.

9. Composition pharmaceutique selon la revendication 8, dans laquelle l'analgésique d'action centrale est choisi parmi la morphine, l'hydromorphone, l'oxymorphone, la méthadone, la mépéridine, l'alfentanil, le fentanyle et le sufentanil.
